(19) Europäisches Patentamt
European Patent Office
Office européen des brevets

(11) **EP 2 481 567 A1**

(12) **EUROPEAN PATENT APPLICATION**
published in accordance with Art. 153(4) EPC

(43) Date of publication:
**01.08.2012 Bulletin 2012/31**

(51) Int Cl.:
***B32B 9/00*** (2006.01)   ***A61F 13/15*** (2006.01)
***A61F 13/514*** (2006.01)   ***A61F 13/551*** (2006.01)

(21) Application number: **10818668.5**

(22) Date of filing: **27.08.2010**

(86) International application number:
**PCT/JP2010/065084**

(87) International publication number:
**WO 2011/036992 (31.03.2011 Gazette 2011/13)**

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**

(30) Priority: **24.09.2009 JP 2009219466**

(71) Applicant: **Unicharm Corporation
Ehime 799-0111 (JP)**

(72) Inventors:
• **KONISHI, Takayoshi
Kanonji-shi
Kagawa 769-1602 (JP)**
• **MIZUTANI, Satoshi
Kanonji-shi
Kagawa 769-1602 (JP)**

(74) Representative: **Knights, Rupert
Saunders & Dolleymore LLP
9 Rickmansworth Road
Watford WD18 0JU (GB)**

(54) **SHEET HAVING AIR PERMEABILITY, WATER DISINTEGRABILITY AND WATER IMPERMEABILITY**

(57)    It is an object of the invention to provide a laminated sheet with air permeability, water disintegratability and water impermeability.

The sheet with air permeability, water disintegratability and water impermeability comprises a base material layer with air permeability and water disintegratability and a hydrophobic fine particle layer, wherein the hydrophobic fine particle layer is situated on the base material layer, and the surface of the hydrophobic fine particle layer on the side that is not in contact with the base material layer has a fine irregular structure formed by layering of the hydrophobic fine particles.

Fig.3

**Description**

Technical Field

The present invention relates to a sheet with air permeability, water disintegratability and water impermeability.

Background Art

Currently, absorbent articles, such as sanitary napkins, fabric absorbent pads and incontinence pads include materials without water disintegratability, and after use they are discarded in waste boxes provided in toilet rooms. The discarded absorbent articles are collected and then disposed. However, when used absorbent articles are inadvertently flushed in flush toilets during disposal, they can potentially clog the pipes of the flush toilets. Research has therefore been conducted on absorbent articles made of water disintegratable materials, that can be flushed in flush toilets directly after use.

PTL 1 discloses a hygienic pad comprising a front sheet, a body fluid absorber and a back sheet, wherein the back sheet is composed of a water disintegratable base material, a water-soluble resin layer and a water-repellent layer, in that order from the outer side.
However, the absorbent article disclosed in PTL 1 has a water-soluble resin layer and water-repellent layer layered with their water-soluble resin layers facing inward, and therefore the water-soluble resin layers serve as barrier layers, virtually no air permeability or moisture permeability is exhibited, and the article very easily becomes musty. Furthermore, because the stiffness of the absorbent article is increased by the water-soluble resin layer, the hand quality is poor.

Also, since the absorbent article of PTL 1 has water-repellency on only one side of the water disintegratable base material, it is able to exhibit leakage resistance for fluid from the water-repellent layer side, such as body fluid absorbed by the absorber, but when fluid has diffused from the water disintegratable base material side, leakage resistance cannot be exhibited when, for example, perspiration has diffused from the underwear side.
In addition, since the absorbent article of PTL 1 is produced by coating a water-soluble resin film with a curable release agent and curing it, it requires a water-soluble resin film-attachment step, a release agent-coating step and a release agent-curing step, and this lowers the production efficiency and increases cost.

Furthermore, since the release agent chemically bonds with the water-soluble resin upon curing in the absorbent article of PTL 1, the dissolution rate of the resin tends to be reduced. Thus, the sections in which the release agent is integrated become fragmented upon water disintegration, and tend to float up in the clarification tank.

Citation List

Patent Literature

PTL 1 Japanese Unexamined Patent Publication No. 2001-333929

Summary of Invention

Technical Problem

The present invention has been accomplished in light of these circumstances, and its object is to solve the problems described above, and specifically to provide a laminated sheet with air permeability, water disintegratability and water impermeability.

Solution to Problem

The present inventors have conducted much diligent research directed toward solving these problems, and as a result we have completed this invention upon finding that the problems can be solved by a sheet having air permeability, water disintegratability and water impermeability, comprising a base material layer with air permeability and water disintegratability and a hydrophobic fine particle layer, wherein the hydrophobic fine particle layer is situated on the base material layer, and the surface of the hydrophobic fine particle layer on the side that is not in contact with the base material layer has a fine irregular structure formed by layering of the hydrophobic fine particles.

Specifically, the present invention relates to the following aspects.

[Aspect 1]

A sheet having air permeability, water disintegratability and water impermeability, comprising a base material layer with air permeability and water disintegratability and a hydrophobic fine particle layer,
wherein the hydrophobic fine particle layer is situated on the base material layer, and
the surface of the hydrophobic fine particle layer on the side that is not in contact with the base material layer has a fine irregular structure formed by layering of the hydrophobic fine particles.

[Aspect 2]

A sheet according to aspect 1, wherein the mean particle size of the hydrophobic fine particles is 1-100 nm.

[Aspect 3]

A sheet according to aspect 1 or 2, wherein the basis weight of the hydrophobic fine particles is 0.1-3 g/m$^2$ .

[Aspect 4]

A sheet according to any one of aspects 1 to 3, wherein the water contact angle on the hydrophobic fine particle layer side is 150° or greater.

[Aspect 5]

A sheet according to any one of aspects 1 to 4, wherein the water pressure resistance from the hydrophobic fine particle layer side is 30 mm or greater.

[Aspect 6]

A sheet according to any one of aspects 1 to 5, wherein the dispersion ratio (%) after a shake-flask test is 60 mass% or greater.

[Aspect 7]

A sheet according to any one of aspects 1 to 6, wherein the air-flow resistance value is no greater than 0.5 kPa·s/m.

[Aspect 8]

A sheet according to any one of aspects 1 to 7, wherein the air-flow resistance value of the sheet is a value of no greater than 1.3 times the air-flow resistance value of the base material.

[Aspect 9]

A sheet according to any one of aspects 1 to 8, wherein the hydrophobic fine particles are inorganic fine particles.

[Aspect 10]

A sheet according to any one of aspects 1 to 9, wherein the hydrophobic fine particles are silica fine particles.

[Aspect 11]

A method for producing a sheet having air permeability, water disintegratability and water impermeability, comprising a base material layer with air permeability and water disintegratability and a hydrophobic fine particle layer, comprising the steps of:

coating a hydrophobic fine particle-containing solution onto the base material having air permeability and water disintegratability, and
drying the coated base material,

wherein the hydrophobic fine particle layer is situated on the base material layer, and
the surface of the hydrophobic fine particle layer on the side that is not in contact with the base material layer has a fine irregular structure formed by layering of the hydrophobic fine particles.

Advantageous Effects of Invention

The sheet of the invention has water impermeability because the hydrophobic fine particle layer has a fine irregular structure formed by layering hydrophobic fine particles, and it has air permeability because the hydrophobic fine particles do not block the base material.
Also, while the sheet of the invention has such water impermeability, it also has water disintegratability because the hydrophobic fine particle layer easily dissociates when it is rubbed in water.
In addition, when the sheet of the invention, which exhibits a water-repelling effect by adhesion of hydrophobic fine particles onto the surface of a water disintegratable base material by intermolecular force, is flushed in a toilet or the like, some of the hydrophobic fine particles are easily shed from the base material by the force of the water stream and the water disintegratable base material exhibits its original water-absorbing behavior causing it to rapidly settle in the water, and therefore it has an excellent settling property in clarification tanks.
Furthermore, the sheet of the invention has high production efficiency because it can be easily produced by coating a base material with a hydrophobic fine particle-containing solution.

Brief Description of Drawing

Fig. 1 is a schematic diagram of an embodiment of an apparatus for production of a sheet of the invention.
Fig. 2 is a schematic diagram of an embodiment of an apparatus for production of a sheet of the invention.
Fig. 3 is a schematic diagram of a wet paper forming system for inline production of a sheet of the invention.

Description of Embodiments

The present invention will now be explained in greater detail.

«Sheet with air permeability, water disintegratability and water impermeability»

[Base material with air permeability and water disintegratability]

Throughout the present specification, "air permeability" means easy passage of air and moisture. The air permeability may be evaluated, for example, by the air-flow resistance value according to KES method F8-AP1.
The base material with air permeability and water disintegratability to be used for the invention has an air-flow resistance value of preferably no greater than about 0.5 kPa·s/m and more preferably no greater than about 0.3 kPa·s/m, according to KES method F8-AP1. A high air-flow resistance value can cause mustiness when a sheet with air permeability, water disintegratability and water impermeability according to the invention is used in an absorbent article.
Throughout the present specification, "water disintegratability" means a quality of disintegrating in a water stream when flushed in a flush toilet. The base material preferably disperses to a state in which it does not hold its original form, in the shake-flask method.
The procedure for the shake-flask method is as follows.
A 10 cm x 10 cm square sample is placed in a 1000 mL flask containing 800 mL of distilled water and shaken in a shaker (SHKV-200, product of Iwaki) for 30 minutes at a shaking speed of 240 rpm, and the condition of the sample after shaking is visually observed.
In consideration of the water disintegratability of the sheet with air permeability, water disintegratability and water impermeability according to the invention, the base material has a dispersion ratio, after the shake-flask test, of preferably about 60 mass% or greater, more preferably about 80 mass% or greater and most preferably about 90 mass% or greater.
The method of evaluating the dispersion ratio is as follows.
The sample that has been subjected to the shake-flask test is filtered with a 2-mesh wire mesh (filament diameter: 1.5 mm, aperture: 11.2 mm, space factor: 77.8%), and calculation is performed by the following formula:

$$\text{Dispersion ratio (\%)} = 100 \times (A-B)/A,$$

where A is the dry mass of the sheet before the test and B is the dry mass of the sheet fibers remaining on the wire mesh.

The base material is not particularly restricted so long as it is a base material satisfying the aforementioned conditions of air permeability and water disintegratability, and examples include fibers, such as pulp, rayon, cupra, cotton, hemp, wool, silk, polyvinyl alcohol, polyethylene, polypropylene, polyester, nylon and vinylon, and nonwoven fabrics composed of such fibers.

The fibers are preferably water-dispersible, from the viewpoint of water disintegratability. Also from the viewpoint of water disintegratability, the fibers preferably have fiber lengths of no greater than about 20 mm.

There are no particular restrictions on the method of forming the nonwoven fabric, and for example, the production may be by a method of using a wet paper machine with high-pressure water jet treatment, or a method of using a cylinder paper machine or cylinder former paper machine, as described in Japanese Unexamined Patent Publication HEI No. 9-228214 or Japanese Unexamined Patent Publication No. 2003-119654.

Also, the base material may be one wherein the fibers are bound by a water-soluble binder, with no particular restrictions on the water-soluble binder, examples of which include polyvinyl alcohol and cellulose derivatives, such as methylcellulose, hydroxyethylcellulose, starch, sodium alginate, sodium polyacrylate and polyacrylic acid esters.

[Hydrophobic fine particle layer]

Throughout the present specification, "hydrophobic fine particles" refers to fine particles of a type such that the hydrophobic fine particle layer with a fine irregular structure formed by layering of the hydrophobic fine particles satisfies the condition of water-repellency explained hereunder. There are no particular restrictions on the hydrophobic fine particles, and for example, they may be inorganic fine particles, such as titanium oxide, zinc oxide, barium sulfate, boron nitride, $SnO_2$, silica, $Cr_2O_3$, $Al_2O_3$, $Fe_2O_3$, SiC, cerium oxide or the like. The surface properties, including the polarity, of the hydrophobic fine particles is particularly important, and surface treatment and surface modification may be carried out as necessary, so that the hydrophobic fine particle layer satisfies the water-repellency property described hereunder.

Fine particles of silica, titanium oxide or zinc oxide are preferred as the hydrophobic fine particles to be used for the invention, especially from the viewpoint of safety. Examples of commercially available hydrophobic fine particles include the ADESSO WR Series by Nicca Chemical Co., Ltd.

The mean particle size of the hydrophobic fine particles is between about 1 nm and about 100 nm, more preferably between about 10 nm and about 80 nm, and even more preferably between about 20 nm and about 60 nm. By using hydrophobic fine particles with this range of mean particle size, it is possible to prevent aggregation and improve the dispersion stability. A mean particle size of less than 1 nm will tend to increase production cost, reduce the thickness of the hydrophobic fine particles on the sheet of the invention, and make it difficult to ensure the water impermeability explained hereunder. A mean particle size of greater than about 100 nm will tend to promote formation of gaps between the particles, make it difficult to ensure water impermeability, promote dissociation of the hydrophobic fine particles from the base material, and result in a harder feel during wearing.

Throughout the present specification, "mean particle size" refers to the value obtained by random pickup of approximately 300 particles from an image taken by an electron microscope, measuring the particle sizes, and calculating the arithmetic mean.

The hydrophobic fine particle layer has a fine irregular structure formed by layering of the hydrophobic fine particles on the surface that is not in contact with the base material layer, i.e. the surface that is to exhibit water impermeability. This is because a hydrophobic fine particle layer with a fine irregular structure will have a larger water contact angle than with a flat surface. The reason for this is as follows.

In a specific base material with a flat surface, the following formula (1) :

$$\cos\theta_r = r\cos\theta \quad (1)$$

is established, where $\theta$ is the fluid contact angle, and $\theta_r$ is the fluid contact angle when the surface area of the material is increased by a factor of r.

Since $1 < r$ in formula (1), if $90° < \theta$, increasing r increases $\theta_r$, or in other words, it increases the fluid contact angle. Incidentally, when $\theta < 90°$, a greater value for r results in a smaller $\theta_r$, or in other words, it decreases the fluid contact angle.

Therefore, if the hydrophobic fine particle layer to be used for the invention has a fine irregular structure, the contact angle for fluids, such as water is increased compared to a flat structure for the hydrophobic fine particle layer. In addition, the fine irregular structure is preferably such that the surface area is further increased, or in other words, such that r in formula (1) is increased.

Examples of fine irregular structures with larger surface areas include fractal structures, such as fine protrusion-like fractal structures.

[Sheet with air permeability, water disintegratability and water impermeability]

In a sheet with air permeability, water disintegratability and water impermeability according to the invention, the base material layer preferably has a basis weight of about 10 to about 100 g/m². A basis weight of less than about 10 g/m² will tend to weaken the strength of the sheet itself and render coating difficult, while a basis weight of greater than about 100 g/m² will tend to increase the density and result in inferior air permeability and water disintegratability.

The hydrophobic fine particle layer in the sheet of the invention has a basis weight of preferably between about 0.1 g/m² and about 3 g/m², and more preferably between about 0.1 g/m² and about 2 g/m². If the basis weight is less than about 0.1 g/m², variation may occur in the hydrophobic fine particles, and locations without water impermeability may be produced. A basis weight of greater than about 3 g/m² will tend to be economically disadvantageous. The height of the hydrophobic fine particle layer is preferably no greater than about 1 $\mu$m.

The air permeability of the sheet of the invention retains the air permeability of the base material mentioned above, i.e. it has an air-flow resistance value of preferably no greater than about 0.5 kPa·s/m and more preferably no greater than about 0.3 kPa·s/m, according to KES method F8-AP1. A high air-flow resistance value can cause mustiness during wearing, when the sheet of the invention is used in an absorbent article. The air-flow resistance value of the sheet of the invention is preferably a value of no greater than about 1.3 times and more preferably a value of no greater than about 1.1 times, the air-flow resistance value of the base material. For example, a value of no greater than about 1.3 times the air-flow resistance value of the base material is an air-flow resistance value of no greater than 0.13 kPa·s/m, if the air-flow resistance value of the base material is 0.1 kPa·s/m.

The water disintegratability of the sheet of the invention is such that it has a dispersion ratio, after a shake-flask test, of preferably about 60 mass% or greater, more preferably about 70 mass% or greater and most preferably about 80 mass% or greater. This is because a dispersion ratio of less than 60 mass% after a shake-flask test can potentially result in clogging of pipes after toilet flushing.

Throughout the present specification, "water impermeability" refers to a property of not allowing passage of water. The water impermeability is imparted by the hydrophobic fine particle layer. The water impermeability can be evaluated based on the water-repellency and/or water resistance. The water-repellency and water resistance are, as a rule, the values obtained by testing from the hydrophobic fine particle layer side of the sheet of the invention.

The water-repellency can be evaluated by the water contact angle, and the water contact angle is preferably greater than about 90°, more preferably about 140° or greater, and even more preferably about 150° or greater. A larger water contact angle results in a higher water-repelling property. The water contact angle can be measured with a Model CA-V automatic contact angle meter by Kyowa Interface Science Co., Ltd., for example. The water resistance can be evaluated by measuring the water pressure resistance according to the water penetration test method A of JIS L1092 (low hydraulic pressure test), and the water pressure resistance is preferably about 30 mm or greater, more preferably about 50 mm or greater and even more preferably about 70 mm or greater.

The mechanism by which the sheet of the invention has air permeability and water disintegratability and also water impermeability is believed to be as follows.

(1) By coating the hydrophobic fine particles onto the surface of the base material with air permeability and water disintegratability, the hydrophobic fine particles create a fine irregular structure formed by layering of the hydrophobic fine particles, such as a surface with a fractal structure.

(2) By forming a fine irregular structure by the hydrophobic fine particle layer surface, the sheet of the invention is imparted with water-repellency (a water contact angle of 90° or greater) or super-water-repellency (a water contact angle of 150° or greater), and the sheet of the invention exhibits water impermeability. Furthermore, since the hydrophobic fine particles are situated on the base material layer, the base material is not clogged and the air permeability of the sheet of the invention is not hindered.

(3) The hydrophobic fine particles are attached by intermolecular force on the surface of the base material, such as the fibers, and therefore when the sheet of the invention is flushed in a flush toilet, the hydrophobic fine particles easily dissociate by the water stream.

(4) When the hydrophobic fine particle layer has disintegrated to some extent, the sheet of the invention exhibits the properties of the water disintegratable base material, and is disintegrated in the water.

Examples for the sheet of the invention include 2-layer sheets (one-side coated sheets) comprising a base material layer with air permeability and water disintegratability and a hydrophobic fine particle layer on the base material layer, and 3-layer sheets (double-side coated sheets) comprising a hydrophobic fine particle layer, a base material layer with air permeability and water disintegratability and another hydrophobic fine particle layer. Various forms may be selected depending on the purpose.

A production apparatus and production method for the sheet of the invention will now be described with reference to the accompanying drawings. It should be noted that the embodiments depicted in the drawings are examples, and do not restrict the invention in any way.

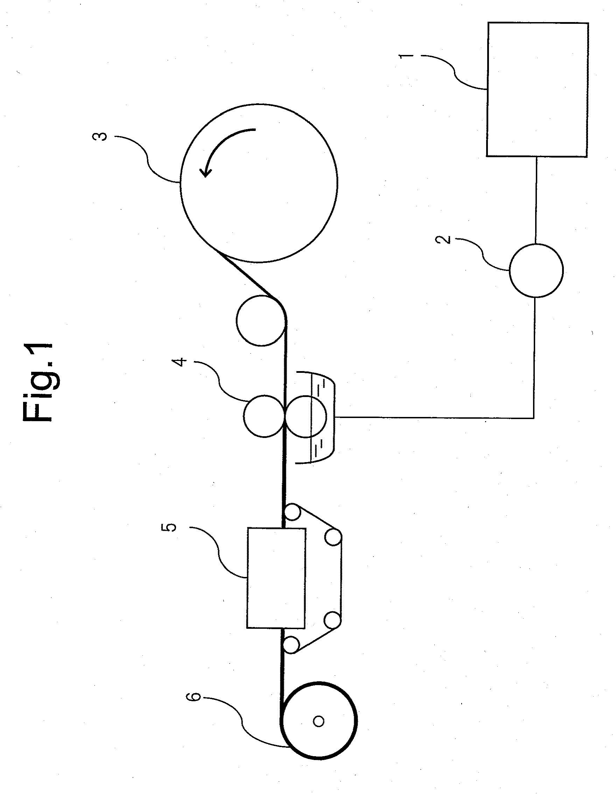

Fig. 1 is a diagram showing an example of an apparatus for production of a sheet of the invention, by single-sided coating of a hydrophobic fine particle-containing solution on a base material with air permeability and water disintegratability. In Fig. 1, a gravure coater 4 is used to coat the base material 3 on one side with the hydrophobic fine particle dispersion conveyed from a hydrophobic fine particle dispersion tank 1 by a metering pump 2, the coated base material is dried with a drier 5, and then the formed sheet is taken up with a take-up roll 6.

Coating of the hydrophobic fine particles is not limited to a gravure coater 4, and a spray coater, for example, may be used instead. In the case of a base material with a low basis weight, such as a tissue, the hydrophobic fine particle-containing solution can penetrate into the base material and impart water-repellency to both sides of the sheet, even if coated only on one side.

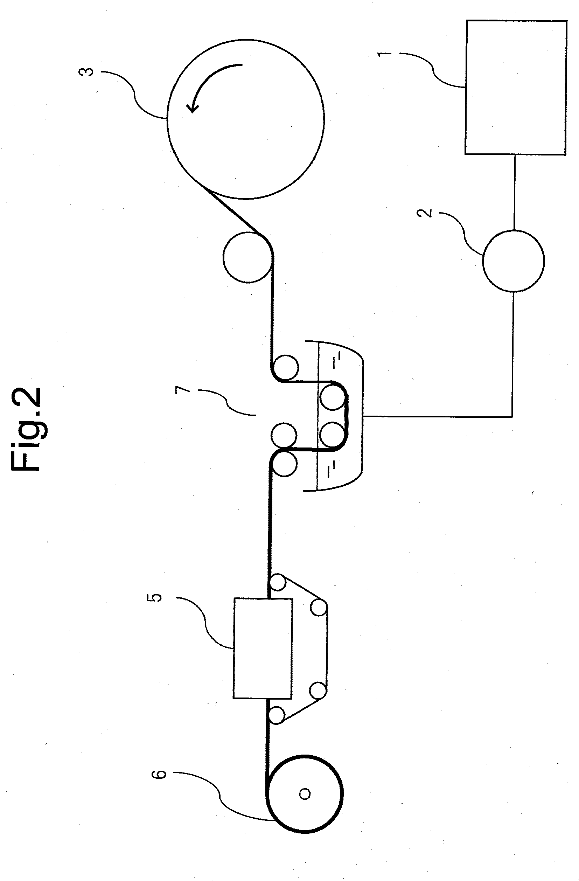

Fig. 2 is a diagram showing an example of an apparatus for production of a sheet of the invention, by double-sided coating of a hydrophobic fine particle-containing solution on a base material with air permeability and water disintegratability. In Fig. 2, a dip nip coater 7 is used to coat the base material 3 onto both sides with the hydrophobic fine particle-containing solution conveyed from a hydrophobic fine particle-containing solution tank 1 by a metering pump 2, the coated base material is dried with a drier 5, and then the formed sheet is taken up with a take-up roll 6. Double-sided coating is useful for imparting water-repellency to both sides of the base material in cases where the base material has a high basis weight and large thickness, such as when it is a water disintegratable nonwoven fabric. For double-sided coating, there may also be used a double-sided coater, spray coater, double-sided gravure coater or the like instead of the dip nip coater mentioned above.

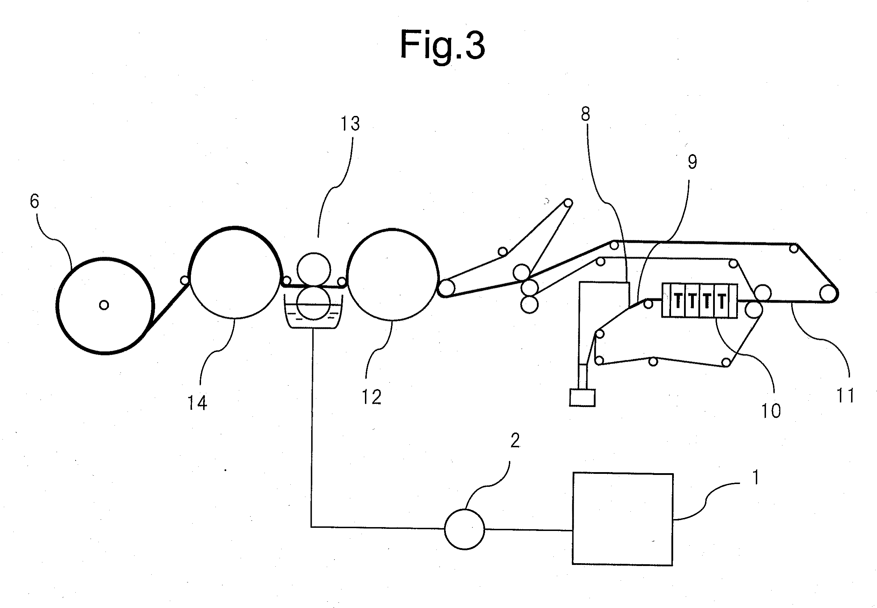

Fig. 3 is a schematic diagram of a wet paper forming system for inline production of a sheet of the invention. In Fig. 3, the starting material for the base material, which is dispersed in water at a prescribed concentration, is conveyed to a web-forming section 8, and a web 9 is formed. The web 9 is then subjected to high-pressure water jet treatment by a high-pressure water jet treatment section 10 to form a treated web 11, which is dried with a primary drier 12. Next, a coating section 13 is used for coating of the dried treated web 11 with the hydrophobic fine particle-containing solution 1 conveyed by the metering pump 2, and drying is performed by a secondary drier 14. After drying, the formed sheet is taken up by a take-up roll 15.

The hydrophobic fine particle-containing solution 1 is preferably a solution comprising the hydrophobic fine particles dispersed in a solvent. Considering the effect on the base, the solvent is preferably a non-water solvent, examples of which include aliphatic alcohols, such as methanol, ethanol, isopropyl alcohol and isobutyl alcohol; ketones, such as acetone and ethyl methyl ketone; esters, such as ethyl acetate; ethers, such as diethyl ether, diisopropyl ether and methyl t-butyl ether; aliphatic hydrocarbons, such as paraffins; alicyclic hydrocarbons, such as cyclohexane; and aromatic hydrocarbons, such as toluene.

There are no particular restrictions on the web-forming section 8, but it is preferably an apparatus employing an inclined short web which allows easy random fiber orientation even for long fibers. There are also no particular restrictions on the high-pressure water jet treatment section 10, and it may be an apparatus sharing a web-forming net, with a high-pressure water jet nozzle mounted on it.

Examples for the coating section 13 include a spray coater and a dip nip coater.

Examples

The present invention will now be explained in detail through the following examples, with the understanding that these examples are not limitative on the invention. Unless otherwise specified, "parts" and "%" refer to parts by mass and mass%, respectively.

[Production Example 1]

A base material starting slurry comprising a mixture of 70 mass% NBKP and 30 mass% rayon fiber (Corona by Daiwabo Co., Ltd., 1.1 dt x 7 mm) was introduced into the web-forming section 8 of the wet paper forming system shown in Fig. 3 to form a web. The formed web was set on web-forming wire and passed through the high-pressure water jet treatment section 10, and subjected to high-pressure water jet treatment from the top side of the web under the conditions shown below, while suctioning the waste water from the bottom side of the wire. The high-pressure water jet-treated web was subjected to press dewatering and drying to obtain a treated web 1 with a basis weight of 40 g/m². A hydrophobic nanosilica paraffin dispersion (ADESSO WR-1 by Nicca Chemical Co., Ltd.) was coated by gravure coating onto the treated web 1, and dried with a hot air dryer (100°C) to obtain a sheet 1 with a basis weight of 0.5 g/m$^2$ of the hydrophobic nanosilica. The mean particle size of the nanosilica was approximately 40 nm.

High-pressure water jet treatment conditions: Four treatments at an energy dose of 0.006324 KW/m$^2$.
Spray nozzle conditions: nozzle diameter: 95 μ, spacing: 0.5 mm pitch
Web-forming wire: LL-70E (double weave) by Nippon Filcon Co., Ltd.

[Production Example 2]

A sheet 2 was obtained according to Production Example 1, except that a hydrophobic nanosilica paraffin dispersion (ADESSO WR-1 by Nicca Chemical Co., Ltd.) was coated to a dry basis weight of 1.0 g/m$^2$.

[Production Example 3]

A sheet 3 was obtained according to Production Example 1, except that a hydrophobic nanosilica paraffin dispersion (ADESSO WR-1 by Nicca Chemical Co., Ltd.) was coated to a dry basis weight of 2.0 g/m$^2$.

[Production Example 4]

A sheet 4 was obtained according to Production Example 1, except that a hydrophobic nanosilica paraffin dispersion (ADESSO WR-1 by Nicca Chemical Co., Ltd.) was coated to a dry basis weight of 3.0 g/m$^2$.

[Production Example 5]

A web 5 (crepe ratio: 7%, basis weight: 15 g/m$^2$) was obtained with a cylinder paper machine from a base material starting slurry with 100 mass% of NBKP. Hydrophobic nanosilica (ADESSO WR-1 by Nicca Chemical Co., Ltd.) was coated by gravure coating onto the web 5 and dried with a hot air dryer (100°C) to obtain a sheet 5 with a basis weight of 1.0 g/m$^2$ of the hydrophobic nanosilica.

[Production Example 6]

A treated web 6 (basis weight: 40.0 g/m$^2$ and then a sheet 6 were obtained in the same manner as Production Example 1, except that a base material starting slurry comprising a mixture of 50 mass% of 1.1 dt x 7 mm rayon fiber (Corona, product of Daiwabo Co., Ltd., 1.1 dt x 7 mm) and 50 mass% of PET fiber (product of Kuraray Co., Ltd., 0.4 dt x 10 mm) was used, and a hydrophobic nanosilica paraffin dispersion (ADESSO WR-1 by Nicca Chemical Co., Ltd.) was coated by gravure coating to a dry basis weight of 1.0 g/m$^2$.

[Comparative Production Example 1]

The treated web 1 produced in Production Example 1 was used as a sheet 7.

[Comparative Production Example 2]

The web 5 produced in Production Example 5 was used as a sheet 8.

[Comparative Production Example 3]

The treated web 6 produced in Production Example 6 was used as a sheet 9.

[Comparative Production Example 4]

One side of the web 5 produced in Production Example 5 (basis weight: 15 g/m$^2$) was laminate-coated with a polyvinyl alcohol (PVA) resin using a T-die (basis weight: 17 g/m$^2$) and further subjected to gravure coating of silicone (UV reactive silicone A/B by T&K Toka Corp.), and the silicone-coated side was exposed to ultraviolet irradiation for UV curing and dried with hot air to obtain a sheet 10. The basis weight of the silicone was 1.0 g/m$^2$.

[Comparative Production Example 5]

A treated web 11 with a basis weight of 40.0 g/m$^2$ was produced by the same procedure as Production Example 1, except for using a base material starting slurry comprising a mixture of 69.7 mass% NBKP, 30 mass% of rayon fiber (Corona by Daiwabo Co., Ltd., 1.1 dt x 7 mm) and 0.3 mass% (solid content) of a sizing agent (styrenemethacrylic acid ester copolymer, SIZEPINE W360 by Arakawa Chemical Industries, Ltd.), and a sheet 11 was obtained.

[Examples 1-6 and Comparative Examples 1-5]

The air permeability, water disintegratability and water impermeability of each of sheets 1 to 11 were evaluated by the following test methods. The results are shown in Table 1.

[Cantilever]

This was measured according to JIS L1096 Bending resistance method A. The values in the MD and CD directions for the sheets of the invention were measured 5 times each, and the arithmetic mean for the total of 10 times was recorded as the cantilever value.
The cantilever value is preferably no greater than 80 mm. A larger value corresponds to greater stiffness, and for example, a more noticeable uncomfortable feeling by the wearer when the sheet is used in an absorbent article.
As used herein, "MD" means the machine direction during production, and "CD" means the cross machine direction perpendicular to the machine direction.

[Water disintegratability]

The shake-flask method was used for evaluation by visual observation and the dispersion ratio value.
The procedure for the shake-flask method is as described above.
The criteria for the visual evaluation were as follows.
G: Dispersion to a state in which the sheet did not retain its original shape.
F: Sheet partially retained its original shape but was mostly dispersed.
P: Sheet retained its original shape.
The dispersion ratio value is the arithmetic mean of 3 measurements.

[Air permeability]

The air-flow resistance value was measured according to KES F8-AP1, and the arithmetic mean of 5 measurements was recorded as the air-flow resistance value.

[Water contact angle]

The water contact angle was measured using a Model CA-V automatic contact angle meter by Kyowa Interface Science Co., Ltd. The water contact angle was determined by measuring the surface of the hydrophobic fine particle layer which was not in contact with the base material layer, and calculating the arithmetic mean.

[Water pressure resistance]

This was measured according to JIS L1092, and the arithmetic mean of 10 measurements was recorded as the water pressure resistance.

[Double-stack water pressure resistance]

Measurement was conducted according to JIS L1092, except for using a stack of 2 samples, and the arithmetic mean of 10 measurements was recorded as the double-stack water pressure resistance.
Table 1

| | | Example 1 | Example 2 | Example 3 | Example 4 | Example 5 | Example 6 | Comp. Ex. 1 | Comp. Ex. 2 | comp. Ex. 3 | Comp. Ex. 4 | Comp. Ex. 5 |
|---|---|---|---|---|---|---|---|---|---|---|---|---|
| Sheet No. | | 1 | 2 | 3 | 4 | 5 | 6 | 7 | 8 | 9 | 10 | 11 |
| Base material composition | NBKP | 70% | 70% | 70% | 70% | 100% | - | 70% | 100% | - | 100% | 69.7% |
| | Rayon | 30% | 30% | 30% | 30% | - | 50% | 30% | - | 50% | - | 30.0% |
| | PET | - | - | - | - | - | 50% | - | - | 50% | - | 0.3% |
| | Sizing agent | - | - | - | - | - | - | - | - | - | - | 0.30 |
| Base material content | g/m$^2$ | 40 | 40 | 40 | 40 | 15 | 40 | 40 | 15 | 40 | 15 | 40 |
| Hydrophobic nanosilica content | g/m$^2$ | 0.50 | 1.00 | 2.00 | 3.00 | 1.00 | 1.00 | - | - | - | - | - |
| PVA resin content | g/m$^2$ | - | - | - | - | - | - | - | - | - | 17.0 | - |
| Silicone content | g/m$^2$ | - | - | - | - | - | - | - | - | - | 1.0 | |
| Sheet thickness | mm | 0.32 | 0.32 | 0.32 | 0.32 | 0.09 | 0.38 | 0.32 | 0.09 | 0.38 | 0.11 | 0.32 |
| Sheet density | g/m$^3$ | 0.127 | 0.128 | 0.131 | 0.134 | 0.178 | 0.108 | 0.125 | 0.167 | 0.105 | 0.300 | 0.125 |
| Cantilever | mm | 72 | 73 | 73 | 75 | 33 | 53 | 68 | 33 | 52 | 93 | 69 |
| Water disintegratability (shake flask method) | Visual | G | G | G | F | G | G | G | G | G | F | P |
| | Dispersion ratio (%) | 92 | 86 | 82 | 65 | 100 | 71 | 98 | 100 | 78 | 58 | 34 |
| Air permeability | Air-flow resistance value (kPa·S/m) | 0.126 | 0.121 | 0.127 | 0.132 | 0.135 | 0.102 | 0.129 | 0.130 | 0.098 | _1) | 0.129 |

| | | Example 1 | Example 2 | Example 3 | Example 4 | Example 5 | Example 6 | Comp. Ex. 1 | Comp. Ex. 2 | comp. Ex. 3 | Comp. Ex. 4 | Comp. Ex. 5 |
|---|---|---|---|---|---|---|---|---|---|---|---|---|
| Water repellency | Water contact angle (°) | 150 | 155 | 157 | 157 | 155 | 157 | 0 | 0 | 0 | 140[2] | 80 |
| | Water pressure resistance (mm) | 91 | 127 | 140 | 141 | 33 | 156 | 2 | 0 | 3 | 350[2] | 45 |
| | Double-stack water pressure resistance (mm) | 286 | 337 | 380 | 375 | 122 | 404 | 5 | 0 | 8 | - | 107 |

1) High air-flow resistance, unmeasurable.
2) Value measured from silicone side.

The sheets of Examples 1 to 4 had air permeability and water disintegratability equivalent to the sheet of Comparative Example 1, which lacked hydrophobic fine particles, while also having water impermeability. In addition, the air-flow resistance values of the sheets of Examples 1 to 4 were virtually unchanged compared to the original base material (the sheet of Comparative Example 1).
Furthermore, the sheets of Examples 1 to 4 had water impermeability at least equivalent to that of the sheet of Comparative Example 5 which employed a sizing agent, and also high water disintegratability.

Example 5 and Comparative Example 2 demonstrate that a sheet with air permeability, water disintegratability and water impermeability can be obtained by coating of hydrophobic fine particles, even when the base material is a water disintegratable tissue using NBKP. Also, Example 6 and Comparative Example 3 demonstrate that a sheet with air permeability, water disintegratability and water impermeability can be obtained by coating of hydrophobic fine particles, even when rayon and PET are used as base materials.

On the other hand, the sheet of Comparative Example 4, which corresponds to the back sheet disclosed in PTL 1, had absolutely no air permeability, and a very large cantilever value.

Industrial Applicability

The sheet with air permeability, water disintegratability and water impermeability according to the invention can be used in sanitary products, such as sanitary napkins and panty liners, sanitary materials, such as disposable diapers, urine leakage-preventing sheets, urine-absorbing pads for incontinent patients, body fluid/blood-absorbing medical goods, wound-dressing materials, cosmetic pack materials, animal excrement-treating materials, agricultural and gardening products, freshness-keeping materials for foods, moisture condensation-proof materials and articles to be used in locations that require moisture absorption and/or moisture retention.

References Signs List

1    Hydrophobic fine particle-containing solution

2    Metering pump

3    Base material

4    Gravure coater

5    Drier

6    Take-up roll

7    Dip nip coater

8    Web-forming section

9    Web

10    High-pressure water jet treatment section

11    Treated web

12    Primary drier

13    Coating section

14    Secondary drier

**EP 2 481 567 A1**

**Claims**

1. A sheet with air permeability, water disintegratability and water impermeability, comprising a base material layer with air permeability and water disintegratability and a hydrophobic fine particle layer,
wherein the hydrophobic fine particle layer is situated on the base material layer, and
the surface of the hydrophobic fine particle layer on the side that is not in contact with the base material layer has a fine irregular structure formed by layering of the hydrophobic fine particles.

2. The sheet according to claim 1, wherein the mean particle size of the hydrophobic fine particles is 1-100 nm.

3. The sheet according to claim 1 or 2, wherein the basis weight of the hydrophobic fine particles is 0.1-3 g/m$^2$.

4. The sheet according to any one of claims 1 to 3, wherein the water contact angle on the hydrophobic fine particle layer side is 150° or greater.

5. The sheet according to any one of claims 1 to 4, wherein the water pressure resistance from the hydrophobic fine particle layer side is 30 mm or greater.

6. The sheet according to any one of claims 1 to 5, wherein the dispersion ratio (%) after a shake-flask test is 60 mass% or greater.

7. The sheet according to any one of claims 1 to 6, wherein the air-flow resistance value is no greater than 0.5 kPa·s/m.

8. The sheet according to any one of claims 1 to 7, wherein the air-flow resistance value of the sheet is a value of no greater than 1.3 times the air-flow resistance value of the base material.

9. The sheet according to any one of claims 1 to 8, wherein the hydrophobic fine particles are inorganic fine particles.

10. The sheet according to any one of claims 1 to 9, wherein the hydrophobic fine particles are silica fine particles.

11. A method for producing a sheet having air permeability, water disintegratability and water impermeability, comprising a base material layer with air permeability and water disintegratability and a hydrophobic fine particle layer, comprising the steps of:

coating a hydrophobic fine particle-containing solution onto the base material having air permeability and water disintegratability, and
drying the coated base material,
wherein the hydrophobic fine particle layer is situated on the base material layer, and
the surface of the hydrophobic fine particle layer on the side that is not in contact with the base material layer has a fine irregular structure formed by layering of the hydrophobic fine particles.

Fig.1

# Fig.2

EP 2 481 567 A1

# Fig.3

EP 2 481 567 A1

| | INTERNATIONAL SEARCH REPORT | International application No. |
|---|---|---|
| | | PCT/JP2010/065084 |

A.   CLASSIFICATION OF SUBJECT MATTER
*B32B9/00*(2006.01)i, *A61F13/15*(2006.01)i, *A61F13/514*(2006.01)i, *A61F13/551*
(2006.01)i

According to International Patent Classification (IPC) or to both national classification and IPC

B.   FIELDS SEARCHED

Minimum documentation searched (classification system followed by classification symbols)
B32B9/00, A61F13/15, A61F13/514, A61F13/551

Documentation searched other than minimum documentation to the extent that such documents are included in the fields searched
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)

C.   DOCUMENTS CONSIDERED TO BE RELEVANT

| Category* | Citation of document, with indication, where appropriate, of the relevant passages | Relevant to claim No. |
|---|---|---|
| A | JP 2003-251759 A  (Keiwa Inc.),<br>09 September 2003 (09.09.2003),<br>entire text<br>(Family: none) | 1-11 |
| A | JP 2006-505476 A  (Degussa AG.),<br>16 February 2006 (16.02.2006),<br>entire text<br>& US 2006/0049376 A1    & WO 2004/039909 A1 | 1-11 |
| A | JP 2007-238931 A  (Nicca Chemical Co., Ltd.),<br>20 September 2007 (20.09.2007),<br>entire text<br>(Family: none) | 1-11 |

| ☒ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 16 September, 2010 (16.09.10) | 28 September, 2010 (28.09.10) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

| International application No. |
| --- |
| PCT/JP2010/065084 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | JP 2008-50380 A  (SNT Co.),<br>06 March 2008 (06.03.2008),<br>entire text<br>(Family: none) | 1-11 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001333929 A
- JP 9228214 A
- JP 2003119654 A